# EUROPEAN PATENT SPECIFICATION

(11) **EP 3 117 265 B1**
(45) Date of publication and mention of the grant of the patent: **29.09.2021**
(21) Application number: 14885797.2
(22) Date of filing: 11.03.2014
(51) Int. Cl.: G02C 7/04, G06K 9/00, G06F 17/00, A61B 3/12, A61B 5/1171

(54) **CONTACT LENSES**
KONTAKTLINSEN
LENTILLES DE CONTACT

(43) Date of publication of application: 18.01.2017
(73) Proprietor: Verily Life Sciences LLC, Mountain View, CA 94043 (US)
(72) Inventor: OTIS, Brian, Mountain View, CA 94043 (US); PARVIZ, Babak, Mountain View, CA 94043 (US)
(74) Representative: Mewburn Ellis LLP
(86) International application number: PCT/US2014/023320
(87) International publication number: WO 2015/137924

(56) References cited:
- EP-A1- 2 508 935
- WO-A2-2014/021602
- JP-A- 2006 302 276
- US-A- 4 014 321
- US-A1- 2006 115 130
- US-A1- 2010 001 926
- US-A1- 2010 142 765
- US-A1- 2011 200 235
- US-A1- 2014 193 045
- US-B1- 6 785 406

## Description

### TECHNICAL FIELD

This disclosure generally relates to iris-scanning contact lenses and biometric identification employing iris scanning contact lenses.

### BACKGROUND

As the interface between humans and technology has grown both in adoption rates and importance in our lives, security has become increasingly important. Verification of users can facilitate security. Biometrics is a class of user verification with the focus of identification of humans using characteristics unique to a particular human. Unfortunately, conventional biometric systems and techniques are relatively inconvenient to use. The inconvenience is exacerbated as the number of secure devices continues to grow. As such, new approaches for user verification are desired.

US 2006/115130 A1 discloses an eyewear display system with an attached or embedded miniaturized display, with user access controlled by biometric identity credentials. Users initially provide biometric (fingerprints, iris, retina, voice, etc.) and/or non-biometric identity credentials to enrol in (and subsequently authenticate themselves to) the eyewear display system. The system also detects when a user physically exits the system, using one or more "presence detection" devices (e.g. optical, acoustic, iris, or retinal presence sensors), thereby ensuring every user is authenticated prior to each session.

### SUMMARY

The following presents a simplified summary of one or more implementations in order to provide a basic understanding of such implementations. This summary is not an extensive overview of all contemplated implementations, and is intended to neither identify key or critical elements of all implementations nor delineate the scope of any or all implementations. Its purpose is to present some concepts of one or more implementations in a simplified form as a prelude to the more detailed description that is presented later.

A contact lens according to a first aspect of the invention is set out in claim 1.

A method facilitating biometric iris recognition according to a second aspect of the invention is set out in claim 7.

A method of biometric identification according to a third aspect of the invention is set out in claim 12.

A system facilitating biometric identification according to a fourth aspect of the invention is set out in claim 15_{.}

Toward the accomplishment of the foregoing and related ends, the one or more implementations include the features hereinafter fully described and particularly pointed out in the claims. The following description and the annexed drawings set forth herein detail certain illustrative aspects of the one or more implementations. These aspects are indicative, however, of but a few of the various ways in which the principles of various implementations can be employed, and the described implementations are intended to include all such aspects and their equivalents.

### BRIEF DESCRIPTION OF THE DRAWINGS

FIG. 1 is an illustration of a block diagram of an exemplary non-limiting system that facilitates iris scanning and/or biometric identification in accordance with implementations described herein.
FIGs. 2A, 2B and 2C are illustrations of block diagrams of exemplary non-limiting contact lenses that facilitate iris scanning in accordance with implementations described herein.
FIG. 3 is an illustration of a block diagram of an exemplary non-limiting circuit that facilitates iris scanning in accordance with implementations described herein.
FIG. 4 is an illustration of a block diagram of an exemplary non-limiting system that facilitates biometric identification in accordance with implementations described herein.
FIG. 5 is an illustration of an exemplary flow diagram of a method that facilitates iris scanning in accordance with implementations described herein.
FIG. 6 is an illustration of an exemplary flow diagram of a method that facilitates biometric identification in accordance with implementations described herein.
FIG. 7 is an illustration of a schematic diagram of an exemplary networked or distributed computing environment for implementing one or more implementations described herein.
FIG. 8 is an illustration of a schematic diagram of an exemplary computing environment for implementing one or more implementations described herein.

### DETAILED DESCRIPTION

Various implementations are now described with reference to the drawings, wherein like reference numerals are used to refer to like elements throughout. In the following description, for purposes of explanation, numerous specific details are set forth in order to provide a more thorough understanding of one or more implementations. It is be evident, however, that such implementations can be practiced without these specific details. In other instances, structures and devices are shown in block diagram form in order to facilitate describing one or more implementations.

It is to be appreciated that in accordance with one or more implementations described in this disclosure, users can opt-out of providing personal information, demographic information, location information, proprietary information, sensitive information, or the like in connection with data gathering aspects. Moreover, one or more implementations described herein can provide for anonymizing collected, received, or transmitted data.

Apparatus, systems and/or methods disclosed herein relate to contact lenses that facilitate iris scanning and/or to biometric iris identification. In particular, a contact lens can include: a transparent substrate formed to cover at least a portion of an iris of an eye; and a circuit. The circuit can include: one or more light sensors disposed on the transparent substrate, that detects light filtered through the iris and incident on the one or more light sensors; a power component, operably coupled to readout circuitry, that supplies power to the readout circuitry; and readout circuitry, operably coupled to the one or more light sensors, that outputs information indicative of the light filtered through the iris and incident on the one or more light sensors. In some implementations, a method of biometric iris identification can include: detecting, at one or more light sensors disposed on a transparent lens covering at least a portion of an eye, light reflected from light incident on an iris of the eye, wherein the light reflected includes image data indicative of a pattern associated with the iris; and outputting an iris fingerprint based in part on the image data.

One or more implementations of the apparatus, systems and/or methods described herein can advantageously facilitate user verification via contact lens biometric iris identification.

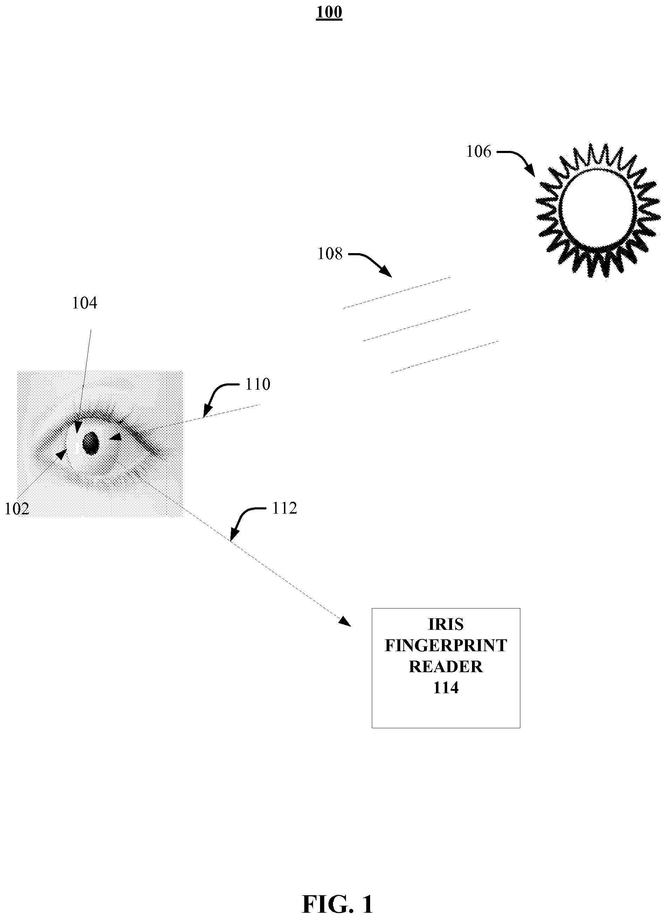

FIG. 1 is an illustration of a block diagram of an exemplary non-limiting system that facilitates iris scanning and/or biometric identification in accordance with implementations described herein. The system 100 includes a contact lens 102 covering at least a portion of an iris 104 and having a circuit, including one or more light sensors (not shown), a power component (not shown) and/or readout circuitry (not shown), disposed on the substrate of the contact lens that performs iris scanning and/or facilitates biometric identification. In some implementations, the system 100 can include the contact lens 102 covering at least a portion of the iris 104 and having the circuit, including one or more light sensors (not shown), a power component (not shown) and/or readout circuitry (not shown), disposed on the substrate of the contact lens that performs iris scanning and/or facilitates biometric identification and an iris fingerprint reader 114. In some implementations, the system 100 can include a light source 106 that emits light rays 108, the contact lens 102 covering at least a portion of the iris 104 and having the circuit, including one or more light sensors (not shown), a power component (not shown) and/or readout circuitry (not shown), disposed on a substrate of the contact lens that performs iris scanning and/or facilitates iris biometric identification, and the iris fingerprint reader 114.

In various implementations, the light source 106 can be the sun, ambient light (e.g., light inside of a building) and/or a specific interrogation light spectra. For example, the interrogation light spectra can be one or more wavelengths to which the light sensors of the contact lens are responsive.

In various implementations, light 110 is received at the iris 104 and is reflected. The one or more light sensors detect the light reflected from, and thus filtered through, the iris. The light filtered through the iris can be information 112 indicative of an iris fingerprint for the wearer of the contact lens 102. In various implementations, the iris fingerprint can be read, processed and/or stored by the iris fingerprint reader 114. The iris fingerprint can also be employed to determine whether access to secure devices can be provided, and/or whether secure information can be downloaded to third-parties, for example.

Accordingly, in an exemplary implementation, the detection surface of the light sensors face towards the iris to receive the light reflected from the iris.

In some implementations, the contact lens can also include a light source on the substrate of the contact lens. The light source could be disposed to project light into the iris of the wearer of the contact lens. The projected light can then be reflected and filtered through the iris and detected by the light sensors. As such, in various implementations, a light source other than ambient light can be employed to project light into the iris of the wearer of the contact lens.

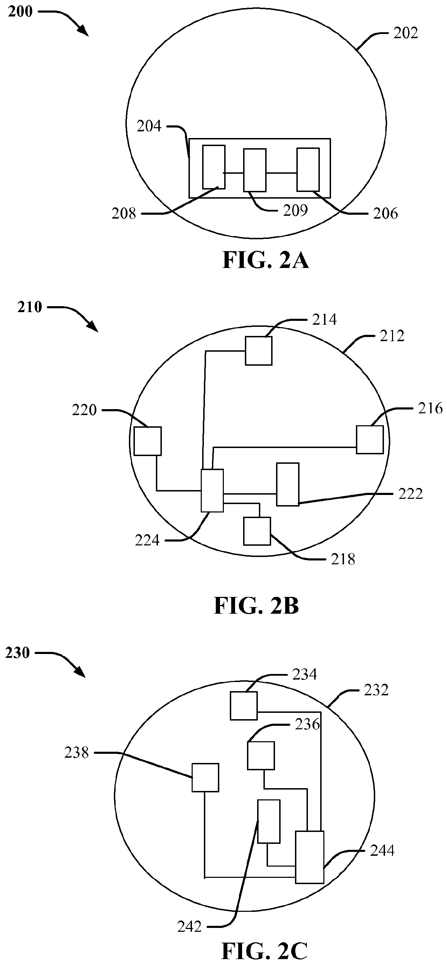

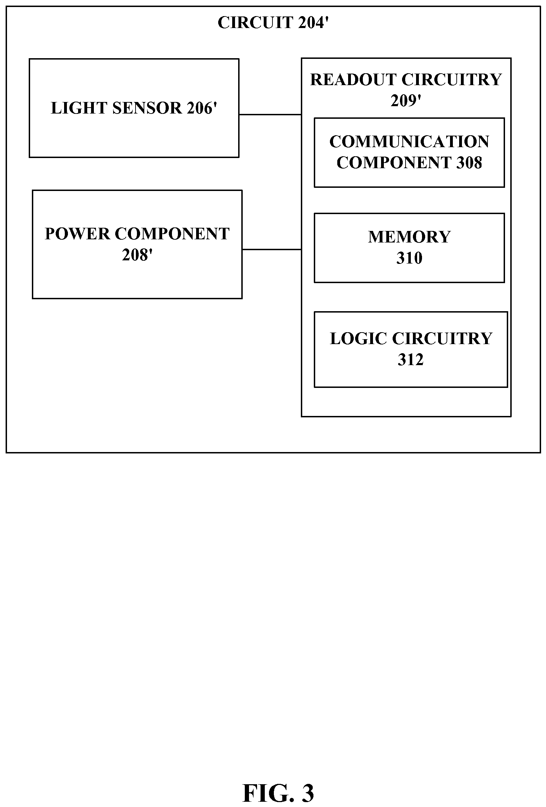

Various implementations of the contact lens will be described with reference to FIGs. 2A, 2B, 2C and 3. FIGs. 2A, 2B and 2C are illustrations of block diagrams of exemplary non-limiting contact lenses that facilitate iris scanning in accordance with implementations described herein. FIG. 3 is an illustration of a block diagram of an exemplary non-limiting circuit that facilitates iris scanning in accordance with implementations described herein.

Turning first to FIG. 2A, the contact lens 200 can include a substrate 202 formed to cover at least a portion of an iris of an eye. In some implementations, the substrate can be transparent, e.g., a transparent polymer. The contact lens 200 can include a circuit 204 disposed on or within the substrate.

The circuit 204 can include at least one light sensor 206, a power component 208 and readout circuitry 209. In various implementations, light sensor 206, power component 208 and/or readout circuitry 209 can be operably, electrically and/or communicatively coupled to one another to perform one or more functions of the circuit. The circuit 204 can be configured to perform iris scanning, generation of an iris fingerprint and/or readout of information (e.g., an iris fingerprint) generated based, at least, on the iris scanning.

In some implementations, the circuit 204 can include a number of chips communicatively and/or electrically coupled to one another and having one or more different functions. For example, a first chip (e.g., the chip associated with power component 208) can be associated with power collection (e.g., harvesting power from incident light), a second chip (e.g., the chip associated with readout circuitry 209) can be associated with communication, and/or a third chip (e.g., the chip associated with light sensor 206) can be associated with detection of light filtered by the iris.

In some implementations, the light sensor 206 can have a detection surface facing the iris of the eye so as to detect light filtered through the iris of the wearer of the contact lens 200, and incident on the light sensor 206.

In some implementations, the light sensor 206 can be a light sensor including complementary metal-oxide-semiconductor (CMOS) components or technology and/or PN-junction components or technology. In some implementations, the light sensor 206 can be a passive structure that generates power upon receiving light (e.g., ambient light or an interrogation wavelength). In some implementations, the light sensor can include organic thin film components and/or technology, carbon nanotube devices and/or technology, thin cell batteries and/or high-density capacitors.

In some implementations, the light sensor 206 can be a transparent and/or translucent or semi-transparent/translucent light sensor. In various implementations, the light sensor 206 can be a light sensor adapted to detect specific light wavelengths. In some implementations, the light sensor 206 can be a monochromatic light sensor.

In some implementations, the light sensor 206 can be less than approximately 500 microns wide. In some implementations, the light sensor 206 can be any width capable of being integrated into a contact lens form factor.

In some implementations, the light sensor 206 can have a detection surface facing outward away from the iris as to perform one or more features including, but not limited to, power collection.

The power component 208 can supply power to the readout circuitry 209. In various implementations, the power component 208 can include one or more photovoltaic cells and/or one or more radio frequency (RF) antennas. In other implementations, the power component 208 can contain any of a number of different types of components for collection and/or generation of power.

While the implementation shown in FIG. 2A illustrates a wire connecting the power component 208 and the readout circuitry 209, in some implementations, the power component 208 can supply power to the readout circuitry 209 wirelessly. As such, the illustrated wire need not be included in the circuitry connecting the power component 208 to the readout circuitry 209.

In various implementations, the power component 208 can wirelessly receive power and/or information for supplying power to the readout circuitry 209. For example, the power component 208 can receive and/or process a signal and/or perform power collection according to any number of approaches, including, but not limited to, a near field communication, far field communication, evanescent frequency tunneling, the use of transformers and/or optical transmission.

The readout circuitry 209 can be communicatively and/or electrically coupled to the power component 208 and the light sensor 206. In some implementations, the readout circuitry 209 can determine the information sensed at the light sensor 206 and/or output such information. For example, in various implementations, the readout circuitry 209 can output information indicative of the light filtered through the iris and incident on the light sensor 206.

In various implementations, the information indicative of the light filtered through the iris can include a pattern associated with the iris. For example, with reference to FIG. 1, light 110 can enter the iris and be reflected from the iris as a unique pattern associated with the iris. The pattern (e.g., signature and/or fingerprint) formed by the filtering can be detected by the light sensor 206. The readout circuitry 209 can output an iris fingerprint, which can be the pattern or generated from the pattern.

The readout circuitry 209 can be low power in some implementations. In some implementations, the readout circuitry 209 can periodically determine the filtered light, fingerprint and/or information collected at the light sensor 206.

In some implementations, the readout circuitry 209 can output the information periodically. For example, the readout circuitry 209 can output the information in response to a polling signal received at the readout circuitry.

In some implementations, the readout circuitry 209 can receive a polling signal from a reader that is in proximity to the contact lens (such as iris fingerprint reader 114, described with reference to FIG. 1). For example, the reader can determine that the contact lens is within a particular proximity of the reader and generate a radio frequency (RF) polling signal. In response to receipt of the polling signal, the readout circuitry 209 can output the information obtained from the light sensor 206 and/or information generated by the readout circuitry 209.

In some implementations, the readout circuitry 209 can broadcast the information obtained from the light sensor 206 and/or information generated by the readout circuitry 209 intermittently. For example, in some implementations, the contact lens can awake at periodic intervals of time (e.g., every hour or once a day), or at a specific time, and the readout circuitry 209 can automatically output the information obtained from the light sensor 206 and/or information generated by the readout circuitry 209.

In some implementations, the readout circuitry 209 can output the information obtained from the light sensor 206 and/or information generated by the readout circuitry 209 continually.

In some implementations, the readout circuitry 209 can output the information obtained from the light sensor 206 and/or information generated by the readout circuitry 209 in response to a trigger and/or condition. For example, in various implementations, the readout circuitry 209 can output the information in response to detection that the reader is within a particular RF proximity and/or if a determination is made that a device utilizing the information stored at the readout circuitry 209 is within proximity (e.g., automobile configured to employ the information for locking or unlocking a door, a computer configured to employ the information for providing password access to the computer or the like) and/or if a determination is made that memory at the readout circuitry 209 is full or near full or the like.

In various implementations, the readout circuitry 209 can output the information from the contact lens 202 wirelessly. In various implementations, the information can be formatted and/or output according to a near field communication standard, far field communication standard, optical techniques or the like. In some implementations, the near field communication can be provided via a cellular telephone that can emit a signal to trigger scanning of the contact lens and/or that can emit a polling signal to poll the contact lens and thereby cause the readout circuitry 209 to output the information.

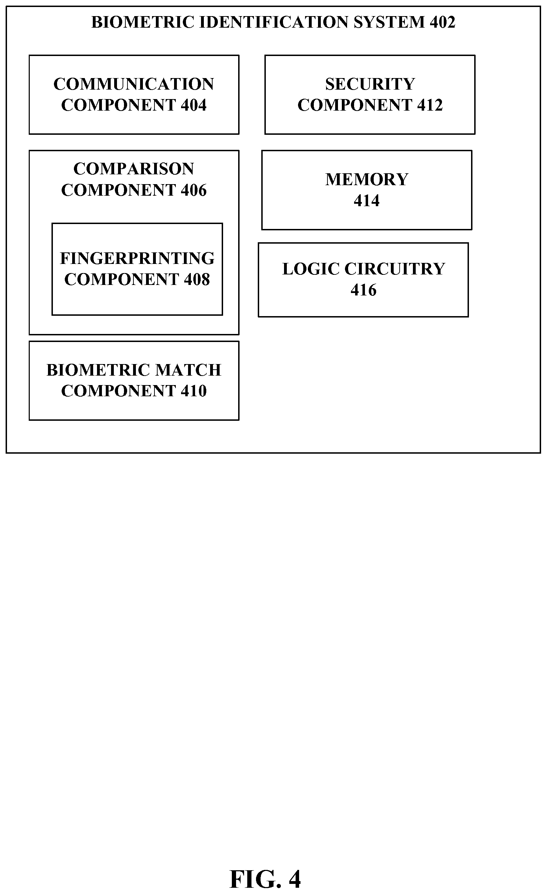

The information output from the readout circuitry 209 can be employed for biometric identification, authentication, as a password or the like. For example, the information can be employed to unlock one or more secure devices (e.g., automobiles, buildings, computers, telephones, safe deposit boxes). In some implementations, the information can be employed to facilitate uploading or downloading information from a cloud-based system. For example, if a biometric identification system (such as that described below with reference to FIG. 4) determines that the information is indicative of a person having access to the cloud-based system, the information can be employed to provide access to the cloud-based system. Additional information stored in the memory of the readout circuitry can be uploaded to the cloud. Similarly, additional information about the person can be downloaded from the cloud to the contact lens and/or to a third-party system if so desired by the person.

While FIG. 2A is described and shown as having a single light sensor 206, in various implementations, the circuit of FIG. 2A can include any number of light sensors. Further, in various implementations, the one or more light sensors, power component 208 and/or readout circuitry 209 can be arranged in any configuration (as shown in FIGs. 2B and 2C).

Turning now to FIG. 2B, a contact lens 210 having a circular array of one or more light sensors is shown. The contact lens 210 can include a substrate 212 formed to cover at least a portion of an iris of an eye. In some implementations, the substrate can be transparent, e.g., a transparent polymer. The contact lens 210 can include a plurality of light sensors 214, 216, 218, 220 arranged in an array on or within the substrate 212. For example, the light sensors 214, 216, 218, 220 can be arranged in a circular array. In other implementations, any number of other types of formations and/or arrays of light sensors 214, 216, 218, 220 can be designed over the substrate 212. While four light sensors are shown, any number of light sensors can be employed as determined by the apparatus and/or system designer. The light sensors 214, 216, 218, 220 can include one or more of the structure and/or functionality of light sensor 206 (and vice versa) described in this disclosure.

The readout circuitry 224 can be electrically and/or communicatively coupled to the light sensors 214, 216, 218, 220 and/or power component 222. As such, the readout circuitry 224 can read the information detected and/or generated by the light sensors 214, 216, 218, 220. The readout circuitry 224 can output the information generated by the light sensors 214, 216, 218, 220 and/or generated by the readout circuitry 224. The readout circuitry 224 can include one or more of the structure and/or functionality of readout circuitry 209 (and vice versa) described in this disclosure.

The power component 222 can provide power to the readout circuitry 224. The power can be provided wirelessly. In various implementations, the power component 222 can receive power via optical and/or RF approaches. The power component 222 can include one or more of the structure and/or functionality of power component 208 (and vice versa) described in this disclosure.

Turning now to FIG. 2C, a contact lens 230 having a group of randomly-placed light sensors is shown. The contact lens 230 can include a substrate 232 formed to cover at least a portion of an iris of an eye. In some implementations, the substrate can be transparent, e.g., a transparent polymer. The contact lens 230 can include a plurality of light sensors 234, 236, 238 arranged in a random or semi-random layout on or within the substrate 212. Certain regions of the substrate may be excluded from potentially including a light sensor, e.g., a region at the center of the substrate. In some implementations, any number of other types of formations and/or arrays of light sensors 234, 236, 238 can be designed over the substrate 232. While three light sensors are shown, any number of light sensors can be employed as determined by the apparatus and/or system designer. The light sensors 234, 236, 238 can include one or more of the structure and/or functionality of light sensor 206 (and vice versa) described in this disclosure.

The readout circuitry 244 can be electrically and/or communicatively coupled to the light sensors 234, 236, 238. As such, the readout circuitry 244 can read the information detected and/or generated by the light sensors 234, 236, 238. The readout circuitry 244 can output the information generated by the light sensors 234, 236, 238 and/or generated by the readout circuitry 244. The readout circuitry 244 can include one or more of the structure and/or functionality of readout circuitry 209 (and vice versa) described in this disclosure.

The power component 242 can provide power to the readout circuitry 244. The power can be provided wirelessly. In various implementations, the power component 242 can receive power via optical and/or RF approaches. The power component 242 can include one or more of the structure and/or functionality of power component 208 (and vice versa) described in this disclosure.

FIG. 3 is an illustration of a block diagram of an exemplary non-limiting circuit that facilitates iris scanning in accordance with implementations described herein. Circuit 204' can be disposed on or within a substrate of a contact lens in various implementations. For example, circuit 204' can be disposed on or within the contact lenses of FIGs. 2A, 2B and/or 2C in various implementations.

In some implementations, the structure and/or functionality of circuit 204 can be as described with reference to circuit 204' of FIG. 3. In some implementations, one or more of the structure and/or functionality of the light sensor 206', power component 208' and/or readout circuitry 209' can have the structure and/or functionality of any of the light sensors, power component and/or readout circuitry described with reference to FIGs. 2A, 2B and 2C. Similarly, the light sensors, power component and/or readout circuitry described with reference to FIGs. 2A, 2B and 2C can have the structure and/or functionality of light sensor 206', power component 208' and/or readout circuitry 209' respectively.

In some implementations, the power component 208' can be coupled to the readout circuitry 209' to provide power to the readout circuitry 209'. The light sensor 206' can be coupled to the readout circuitry 209' to enable communication between the light sensor 206' and the readout circuitry 209'. For example, the readout circuitry 209' can read or detect the light filtered by the iris that is sensed by the light sensor 206'. In some implementations, the readout circuitry 209' can detect and/or generate an iris fingerprint based on the light filtered by the iris and sensed by the light sensor 206'.

In some implementations, the readout circuitry 209' can include a communication component 308 configured to transmit and/or receive information collected and/or generated by the readout circuitry 209'. For example, the communication component 308 can be configured to transmit and/or receive the iris fingerprint and/or other information about the wearer of the contact lens. In some implementations, the other information about the wearer of the contact lens can include passport information, driver's license information, passwords, biographical information, medical information (e.g., for use by medical personnel during a medical emergency), pictures or the like, as may be authorized by the wearer. The information can be transmitted and/or received in response to biometric identification of the wearer of the contact lens. For example, information can be transmitted and/or received to or from a server-based or cloud-based system in response to the successful identification.

In some implementations, the communication component 308 can be configured to transmit the iris fingerprint to a reader of the contact lens located remote from the contact lens.

In some implementations, the communication component 308 can be configured to transmit the iris fingerprint to a secure device to unlock the device. The secure device can include, but is not limited to, an automobile or door lock, computer, telephone, safe deposit box or the like.

The readout circuitry 209' can also include memory 310 and a logic circuitry 312. The memory 310 can be a computer-readable storage medium storing computer-executable instructions and/or information for performing the functions described in this disclosure with reference to the circuit 204'. For example, memory 310 can store computer-executable instructions in some implementations, for performing the functions of iris fingerprint generation, reading out information indicative of light filtered by the iris and detected by the light sensor 206', receiving and/or responding to a polling signal or the like. In some implementations, the memory 310 can store information about the wearer of the contact lens including, but not limited to, biographical information, pictures, passport information, driver's license information, emergency medical information (e.g., allergies or particular medical conditions) or the like, as provided by the wearer. Logic circuitry 312 can perform one or more of the functions described in this disclosure with reference to readout circuitry 209 (or components thereof).

FIG. 4 is an illustration of a block diagram of an exemplary non-limiting system that facilitates biometric identification in accordance with implementations described herein. The biometric identification system 402 can include a communication component 404, a comparison component 406, a fingerprinting component 408, a biometric match component 410, a security component 412, a memory 414 and/or a logic circuitry 416. In various implementations, one or more of the communication component 404, comparison component 406, fingerprinting component 408, biometric match component 410, security component 412, memory 414 and/or logic circuitry 416 can be electrically and/or communicatively coupled to one another.

The communication component 404 can be configured to transmit and/or receive information to or from the contact lens and/or a third-party system. For example, the communication component 404 can transmit information (e.g., the iris fingerprint and/or authorization to unlock a security device or system, a password being transmitted based on a determination by the biometric identification system 402 that the wearer of the contact lens has been authenticated, etc.).

In some implementations, the communication component 404 can upload and/or download information associated with a person wearing the contact lens.

The comparison component 406 can compare a first iris fingerprint with a second iris fingerprint. The first and/or second fingerprint can be stored locally at the biometric identification system 402 (e.g., in the memory 414 of the biometric identification system 402) or remotely at a location other than the biometric identification system (e.g., in the contact lens). In some implementations, the first iris fingerprint can be retrieved from the contact lens and the second iris fingerprint can be a previously-stored fingerprint. The comparison component 406 can include and/or be operably coupled to a fingerprinting component 408.

In some implementations, the fingerprinting component 408 can generate the first and/or second iris fingerprint. The first and/or second fingerprint can be generated based, at least, on an iris pattern retrieved from circuitry coupled to one or more light sensors disposed on or within the contact lens (e.g., the readout circuitry described with reference to FIGs. 2A, 2B, 2C and/or 3).

The biometric match component 410 can determine whether a biometric match exists between the first and second fingerprints. The determination can be based, at least, on the comparison by the comparison component 404.

The security component 412 can lock and/or unlock a device based on the determination by the biometric match component 410 as to whether a match exists. In some implementations, the security component 412 can unlock a device based on a determination that a biometric match exists.

In some implementations, the security component 412 can provide access to another system (not shown) based, at least, on a determination that a biometric match exists. In various implementations, the system to which access is provided can be a cloud-based system, an automobile or other transportation system, a door security system, a computer security system and/or a mobile phone security system.

The memory 414 can be a computer-readable storage medium storing computer-executable instructions and/or information for performing the functions described in this disclosure with reference to the biometric identification system 402. The logic circuitry 416 can perform one or more of the functions described in this disclosure with reference to the biometric identification system 402 (or components thereof).

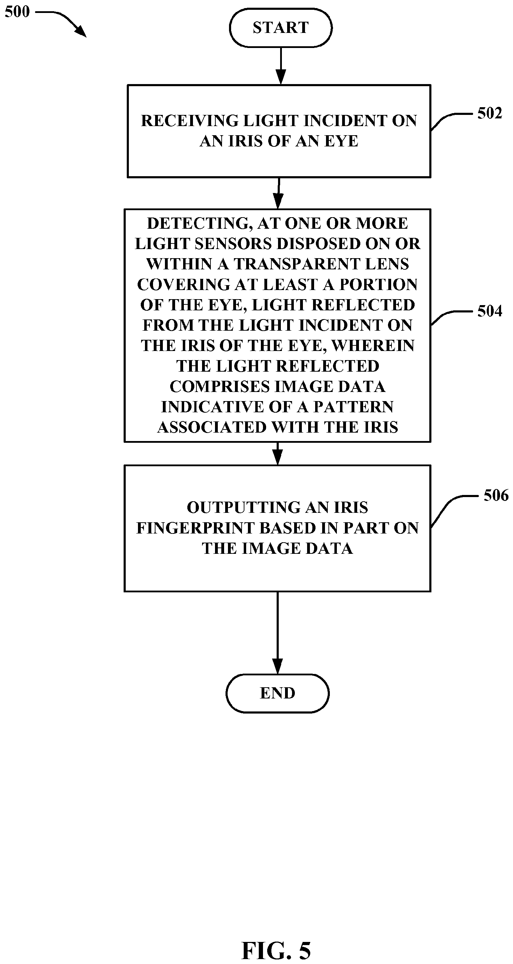

FIG. 5 is an illustration of an exemplary flow diagram of a method that facilitates iris scanning in accordance with implementations described herein. At 502, method 500 can include receiving light incident on an iris of an eye (e.g., using the light sensor 206'). In various implementations, the light incident on the iris can be ambient light and/or a selected interrogation light wavelength.

At 504, method 500 can include detecting, at one or more light sensors disposed on or within a transparent lens covering at least a portion of the eye, light reflected from light incident on the iris of the eye (e.g., using the light sensor 206'). The light reflected can include image data indicative of a pattern associated with the iris.

In some implementations, the one or more light sensors can be a complementary metal oxide semiconductor (CMOS) light sensor or a monochromatic light sensor. For example, in some implementations, the one or more light sensors can be configured to detect light of one or more selected wavelengths.

At 506, method 500 can include outputting an iris fingerprint based in part on the image data (e.g., using the communication component 308 of the readout circuitry 209'). In some implementations, outputting the image data can be performed wirelessly. In some implementations, outputting the image data is performed according to a near field communication standard. For example, a near field communication can be provided via a mobile telephone that can emit a signal to scan the contact lens and/or that can emit a polling signal to poll the contact lens and thereby cause the outputting of the iris fingerprint.

Although not shown, method 500 can also include receiving a polling signal (at the communication component 308 of the readout circuitry 209'). In these implementations, outputting the image data can be performed periodically in response to receiving the polling signal.

Although not shown, method 500 can also include receiving power at the one or more light sensors (e.g., using the light sensor 206'). The power can be received wirelessly in some implementations.

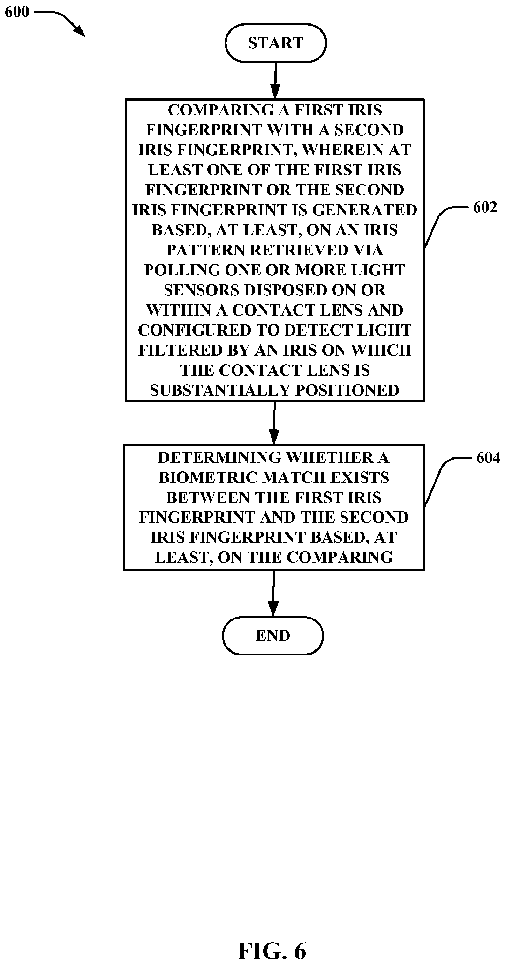

FIG. 6 is an illustration of an exemplary flow diagram of a method that facilitates biometric identification in accordance with implementations described herein.

At 602, method 600 can include comparing a first iris fingerprint with a second iris fingerprint (e.g., using the comparison component 406). In various implementations, the first iris fingerprint or the second iris fingerprint can be generated based, at least, on an iris pattern retrieved via polling one or more light sensors disposed on or within a contact lens. The polling can be performed periodically in some implementations. In some implementations, the polling can be performed over a wireless communication channel according to a near field communication standard.

The first and/or second fingerprints can be generated by the fingerprinting component 406 in some implementations. In some embodiments, the first and/or second fingerprint can be received for comparison with the other fingerprint, which may be stored in a memory or database, for example.

The one or more light sensors can be configured to detect light filtered by an iris on which the contact lens is substantially positioned. In some implementations, the one or more light sensors can be CMOS light sensors.

At 604, method 600 can include determining whether a biometric match exists between the first iris fingerprint and the second iris fingerprint based, at least, on the comparing (e.g., using the biometric match component 410).

One or more of the apparatus, systems and/or methods described herein can be employed in or in association with automotive, consumer electronics and/or security apparatus, systems and/or methods. In various implementations, one or more of the apparatus, systems and/or methods can be employed in systems governed by fingerprinting standards, iris recognition and/or identification standards or the like.

### EXEMPLARY NETWORKED AND DISTRIBUTED ENVIRONMENTS

One of ordinary skill in the art can appreciate that various aspects described in this disclosure can be implemented in connection with any computer or other client or server device, which can be deployed as part of a computer network or in a distributed computing environment, and can be connected to any kind of data store where user verification (e.g., fingerprints) may be found. In this regard, the various implementations described in this disclosure can be implemented in any computer system or environment having any number of memory or storage units, and any number of applications and processes occurring across any number of storage units. This includes, but is not limited to, an environment with server computers and client computers deployed in a network environment or a distributed computing environment, having remote or local storage.

Distributed computing provides sharing of computer resources and services by communicative exchange among computing devices and systems. These resources and services include the exchange of information, cache storage and disk storage for objects, such as files. These resources and services can also include the sharing of processing power across multiple processing units for load balancing, expansion of resources, specialization of processing, and the like. Distributed computing takes advantage of network connectivity, allowing clients to leverage their collective power to benefit the entire enterprise. In this regard, a variety of devices may have applications, objects or resources that may participate in the various implementations of this disclosure.

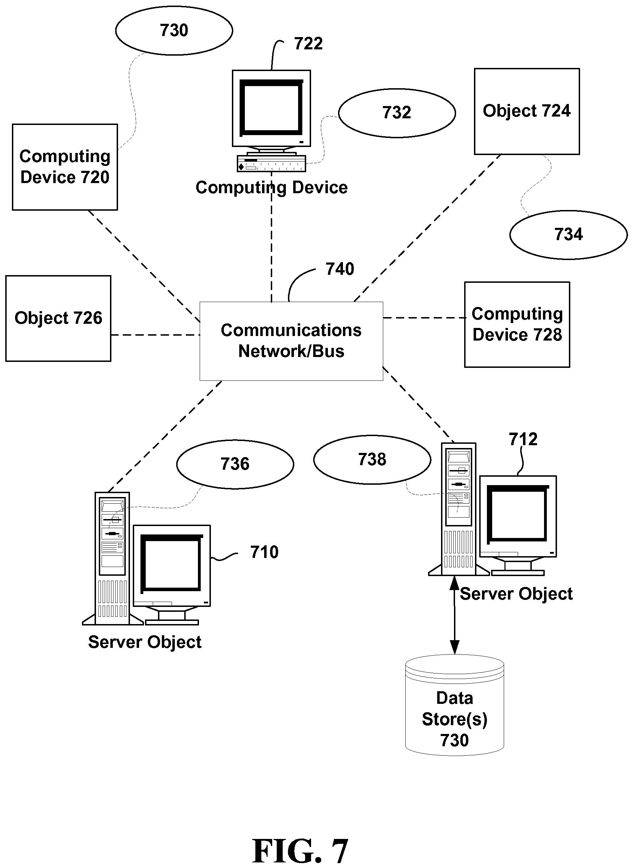

FIG. 7 provides a schematic diagram of an exemplary networked or distributed computing environment in which aspects described in this disclosure can be implemented. The distributed computing environment includes computing objects 710, 712, etc. and computing objects or devices 720, 722, 724, 726, 728, etc., which can include programs, methods, data stores, programmable logic, etc., as represented by applications 730, 732, 734, 736, 738. It can be appreciated that computing objects 710, 712, etc. and computing objects or devices 720, 722, 724, 726, 728, etc. can include different devices, such as personal digital assistants (PDAs), audio/video devices, mobile phones, personal digital music players, personal computers, laptops, tablets, etc.

Each computing object 710, 712, etc. and computing objects or devices 720, 722, 724, 726, 728, etc. can communicate with one or more other computing objects 710, 712, etc. and computing objects or devices 720, 722, 724, 726, 728, etc. by way of the communications network 740, either directly or indirectly. Even though illustrated as a single element in FIG. 7, network 740 can include other computing objects and computing devices that provide services to the system of FIG. 7, and/or can represent multiple interconnected networks, which are not shown. Each computing object 710, 712, etc. or computing objects or devices 720, 722, 724, 726, 728, etc. can also contain an application, such as applications 730, 732, 734, 736, 738, that might make use of an application programming interface (API), or other object, software, firmware and/or hardware, suitable for communication with or implementation of the various implementations of the subject disclosure.

There are a variety of systems, components, and network configurations that support distributed computing environments. For example, computing systems can be connected together by wired or wireless systems, by local networks or widely distributed networks. Currently, many networks are coupled to the Internet, which provides an infrastructure for widely distributed computing and encompasses many different networks, though any network infrastructure can be used for exemplary communications made incident to the systems as described in various implementations.

Thus, a host of network topologies and network infrastructures, such as client/server, peer-to-peer, or hybrid architectures, can be utilized. The client can be a member of a class or group that uses the services of another class or group. A client can be a computer process, e.g., roughly a set of instructions or tasks, that requests a service provided by another program or process. A client can utilize the requested service without having to know all working details about the other program or the service itself.

As used in this application, the terms "component," "component," "system," and the like are intended to refer to a computing-related entity, either hardware, software, firmware, a combination of hardware and software, software and/or software in execution. For example, a component can be, but is not limited to being, a process running on a processor, a processor, an object, an executable, a thread of execution, a program, and/or a computer. By way of illustration, both an application running on a computing device and/or the computing device can be a component. One or more components can reside within a process and/or thread of execution and a component can be localized on one computer and/or distributed between two or more computers. In addition, these components can execute from various computer-readable storage media having various data structures stored thereon. The components can communicate by way of local and/or remote processes such as in accordance with a signal having one or more data packets (e.g., data from one component interacting with another component in a local system, distributed system, and/or across a network such as the Internet with other systems by way of the signal).

Moreover, the term "or" is intended to mean an inclusive "or" rather than an exclusive "or." That is, unless specified otherwise, or clear from the context, the phrase "X employs A or B" is intended to mean any of the natural inclusive permutations. That is, the phrase "X employs A or B" is satisfied by any of the following instances: X employs A; X employs B; or X employs both A and B. In addition, the articles "a" and "an" as used in this application and the appended claims should generally be construed to mean "one or more" unless specified otherwise or clear from the context to be directed to a singular form.

In a client/server architecture, particularly a networked system, a client can be a computer that accesses shared network resources provided by another computer, e.g., a server. In the illustration of FIG. 7, as a non-limiting example, computing objects or devices 720, 722, 724, 726, 728, etc. can be thought of as clients and computing objects 710, 712, etc. can be thought of as servers where computing objects 710, 712, etc. provide data services, such as receiving data from client computing objects or devices 720, 722, 724, 726, 728, etc., storing of data, processing of data, transmitting data to client computing objects or devices 720, 722, 724, 726, 728, etc., although any computer can be considered a client, a server, or both, depending on the circumstances. Any of these computing devices can process data, or request transaction services or tasks that can implicate the techniques for systems as described in this disclosure for one or more implementations.

A server can be typically a remote computer system accessible over a remote or local network, such as the Internet or wireless network infrastructures. The client process can be active in a first computer system, and the server process can be active in a second computer system, communicating with one another over a communications medium, thus providing distributed functionality and allowing multiple clients to take advantage of the information-gathering capabilities of the server. Any software objects utilized pursuant to the techniques described in this disclosure can be provided standalone, or distributed across multiple computing devices or objects.

In a network environment in which the communications network/bus 740 can be the Internet, for example, the computing objects 710, 712, etc. can be Web servers, file servers, media servers, etc. with which the client computing objects or devices 720, 722, 724, 726, 728, etc. communicate via any of a number of known protocols, such as the hypertext transfer protocol (HTTP). Objects 710, 712, etc. can also serve as client computing objects or devices 720, 722, 724, 726, 728, etc., as can be characteristic of a distributed computing environment.

### EXEMPLARY COMPUTING DEVICE

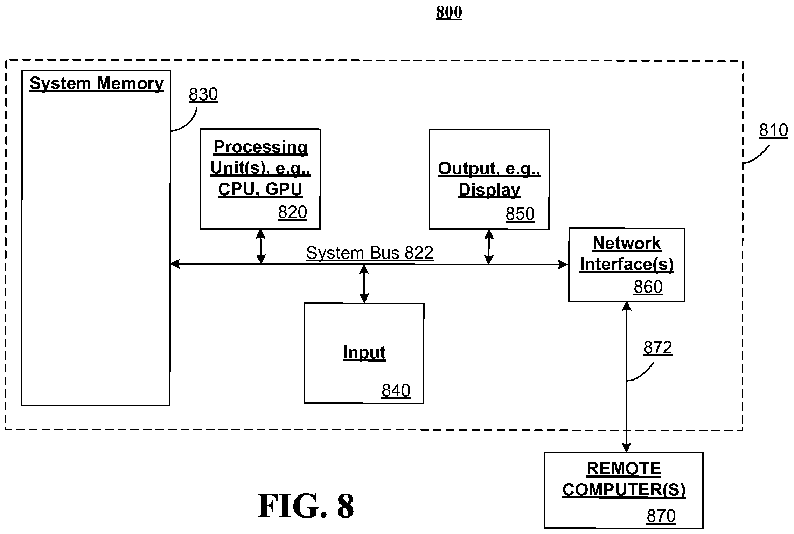

As mentioned, advantageously, the techniques described in this disclosure can be applied to any suitable device. It is to be understood, therefore, that handheld, portable and other computing devices and computing objects of all kinds are contemplated for use in connection with the various implementations, e.g., anywhere that a device may wish to read or write transactions from or to a data store or wherein secure devices may need to be locked, unlocked and/or otherwise accessed, either from locations proximate to or remote from the device. Accordingly, the below remote computer described below in FIG. 8 is but one example of a computing device. Additionally, a suitable server can include one or more aspects of the below computer, such as a user fingerprint server, a biometric identification server or other server components.

Although not required, implementations can be partly implemented via an operating system, for use by a developer of services for a device or object, and/or included within application software that operates to perform one or more functional aspects of the various implementations described in this disclosure. Software can be described in the general context of computer executable instructions, such as program components, being executed by one or more computers, such as client workstations, servers or other devices. Those skilled in the art will appreciate that computer systems have a variety of configurations and protocols that can be used to communicate data, and thus, no particular configuration or protocol is to be considered limiting.

FIG. 8 thus illustrates an example of a suitable computing system environment 800 in which one or aspects of the implementations described in this disclosure can be implemented, although as made clear above, the computing system environment 800 is only one example of a suitable computing environment and is not intended to suggest any limitation as to scope of use or functionality. Neither is the computing environment 800 to be interpreted as having any dependency or requirement relating to any one or combination of components illustrated in the exemplary computing environment 800.

With reference to FIG. 8, an exemplary computing environment 800 for implementing one or more implementations includes a computing device in the form of a computer 810 is provided. Components of computer 810 can include, but are not limited to, a processing unit 820, a system memory 830, and a system bus 822 that couples various system components including the system memory to the processing unit 820.

Computer 810 typically includes a variety of computer readable media and can be any available media that can be accessed by computer 810. The system memory 830 can include computer storage media in the form of volatile and/or nonvolatile memory such as read only memory (ROM) and/or random access memory (RAM). By way of example, and not limitation, memory 830 can also include an operating system, application programs, other program components, and program data.

A user can enter commands and information into the computer 810 through input devices 840, non-limiting examples of which can include a keyboard, keypad, a pointing device, a mouse, stylus, touchpad, touch screen, trackball, motion detector, camera, microphone, joystick, game pad, scanner, video camera or any other device that allows the user to interact with the computer 810. A monitor or other type of display device can be also connected to the system bus 822 via an interface, such as output interface 850. In addition to a monitor, computers can also include other peripheral output devices such as speakers and a printer, which can be connected through output interface 850.

The computer 810 can operate in a networked or distributed environment using logical connections to one or more other remote computers, such as remote computer 880. The remote computer 880 can be a personal computer, a server, a router, a network PC, a peer device or other common network node, or any other remote media consumption or transmission device, and can include any or all of the elements described above relative to the computer 810. The logical connections depicted in FIG. 8 include a network 882, such local area network (LAN) or a wide area network (WAN), but can also include other networks/buses e.g., cellular networks.

As mentioned above, while exemplary implementations have been described in connection with various computing devices and network architectures, the underlying concepts can be applied to any network system and any computing device or system in which it is desirable to publish or consume media in a flexible way.

Also, there are multiple ways to implement the same or similar functionality, e.g., an appropriate API, tool kit, driver code, operating system, control, standalone or downloadable software object, etc. which enables applications and services to take advantage of the techniques detailed herein. Thus, implementations herein are contemplated from the standpoint of an API (or other software object), as well as from a software or hardware object that implements one or more aspects described in this disclosure. Thus, various implementations described in this disclosure can have aspects that are wholly in hardware, partly in hardware and partly in software, as well as in software.

Computing devices typically include a variety of media, which can include computer-readable storage media and/or communications media, in which these two terms are used herein differently from one another as follows. Computer-readable storage media can be any available storage media that can be accessed by the computer, can be typically of a non-transitory nature, and can include both volatile and nonvolatile media, removable and non-removable media. By way of example, and not limitation, computer-readable storage media can be implemented in connection with any method or technology for storage of information such as computer-readable instructions, program components, structured data, or unstructured data. Computer-readable storage media can include, but are not limited to, RAM, ROM, electrically erasable programmable read only memory (EEPROM), flash memory or other memory technology, compact disc read only memory (CD-ROM), digital versatile disk (DVD) or other optical disk storage, magnetic cassettes, magnetic tape, magnetic disk storage or other magnetic storage devices, or other tangible and/or non-transitory media which can be used to store desired information. Computer-readable storage media can be accessed by one or more local or remote computing devices, e.g., via access requests, queries or other data retrieval protocols, for a variety of operations with respect to the information stored by the medium.

On the other hand, communications media typically embody computer-readable instructions, data structures, program components or other structured or unstructured data in a data signal such as a modulated data signal, e.g., a carrier wave or other transport mechanism, and includes any information delivery or transport media. The term "modulated data signal" or signals refers to a signal that has one or more of its characteristics set or changed in such a manner as to encode information in one or more signals. By way of example, and not limitation, communication media include wired media, such as a wired network or direct-wired connection, and wireless media such as acoustic, radio frequency (RF), infrared and other wireless media.

It is to be understood that aspects described in this disclosure can be implemented in hardware, software, firmware, middleware, microcode, or any combination thereof. For a hardware implementation, the processing units can be implemented within one or more application specific integrated circuits (ASICs), digital signal processors (DSPs), digital signal processing devices (DSPDs), programmable logic devices (PLDs), field programmable gate arrays (FPGAs), processors, controllers, micro-controllers, logic circuitry and/or other electronic units designed to perform the functions described in this disclosure, or a combination thereof.

When the aspects are implemented in software, firmware, middleware or microcode, program code or code segments, they can be stored in a machine-readable medium (or a computer-readable storage medium), such as a storage component. A code segment can represent a procedure, a function, a subprogram, a program, a routine, a subroutine, a component, a software package, a class, or any combination of instructions, data structures, or program statements. A code segment can be coupled to another code segment or a hardware circuit by passing and/or receiving information, data, arguments, parameters, or memory contents. Information, arguments, parameters, data, etc. can be passed, forwarded, or transmitted using any suitable means including memory sharing, message passing, token passing, network transmission, etc.

For a software implementation, the techniques described in this disclosure can be implemented with components or components (e.g., procedures, functions, and so on) that perform the functions described in this disclosure. The software codes can be stored in memory units and executed by processors. A memory unit can be implemented within the processor or external to the processor, in which case it can be communicatively coupled to the processor via various structures.

The word "exemplary" is used herein to mean serving as an example, instance, or illustration. For the avoidance of doubt, the subject matter disclosed herein is not limited by such examples. In addition, any aspect or design described in this disclosure as "exemplary" is not necessarily to be construed as preferred or advantageous over other aspects or designs, nor is it meant to preclude equivalent exemplary structures and techniques known to those of ordinary skill in the art. Furthermore, to the extent that the terms "includes," "has," "contains," and other similar words are used in either the detailed description or the claims, for the avoidance of doubt, such terms are intended to be inclusive in a manner similar to the term "comprising" as an open transition word without precluding any additional or other elements.

What has been described above includes examples of one or more implementations. It is, of course, not possible to describe every conceivable combination of components or methodologies for purposes of describing the aforementioned implementations, but one of ordinary skill in the art can recognize that many further combinations and permutations of various implementations are possible. Accordingly, the described implementations are intended to embrace all such alterations, modifications and variations that fall within the spirit and scope of the appended claims. Moreover, use of the term "an implementation" or "one implementation" throughout is not intended to mean the same implementation unless specifically described as such. Further, use of the term "plurality" can mean two or more.

The aforementioned systems have been described with respect to interaction between several components. It can be appreciated that such systems and components can include those components or specified sub-components, some of the specified components or sub-components, and/or additional components, and according to various permutations and combinations of the foregoing. Sub-components can also be implemented as components communicatively coupled to other components rather than included within parent components (hierarchical). Additionally, it is to be noted that one or more components can be combined into a single component providing aggregate functionality or divided into several separate sub-components, and that any one or more middle layers, such as a management layer, can be provided to communicatively couple to such sub-components in order to provide integrated functionality. Any components described in this disclosure can also interact with one or more other components not specifically described in this disclosure but generally known by those of skill in the art.

In view of the exemplary systems described above methodologies that can be implemented in accordance with the described subject matter will be better appreciated with reference to the flowcharts of the various figures. While for purposes of simplicity of explanation, the methodologies are shown and described as a series of blocks, it is to be understood and appreciated that the claimed subject matter is not limited by the order of the blocks, as some blocks can occur in different orders and/or concurrently with other blocks from what is depicted and described in this disclosure. Where nonsequential, or branched, flow is illustrated via flowchart, it can be appreciated that various other branches, flow paths, and orders of the blocks, can be implemented which achieve the same or a similar result. Moreover, not all illustrated blocks can be required to implement the methodologies described in this disclosure after.

In addition to the various implementations described in this disclosure, it is to be understood that other similar implementations can be used or modifications and additions can be made to the described implementation(s) for performing the same or equivalent function of the corresponding implementation(s) without deviating there from. Still further, multiple processing chips or multiple devices can share the performance of one or more functions described in this disclosure, and similarly, storage can be provided across a plurality of devices. The invention is not to be limited to any single implementation, but rather can be construed in the scope in accordance with the appended claims.

## Claims

1. A contact lens (102, 200, 210, 230), comprising:
a transparent substrate (202, 212, 232) formed to cover at least a portion of an iris (104) of an eye; and
a circuit (204) comprising:
one or more light sensors (206, 214, 216, 218, 220, 234, 236, 238) disposed directly on or within the transparent substrate (202, 212, 232) and configured to detect light reflected from, and thus filtered through, the iris (104) and incident on the one or more light sensors (206, 214, 216, 218, 220, 234, 236, 238), wherein the one or more light sensors are configured to detect image data from the reflected light, the image data being indicative of a pattern associated with the iris;
readout circuitry (209, 224, 244), operably coupled to the one or more light sensors (206, 214, 216, 218, 220, 234, 236, 238), configured to output information indicative of the pattern associated with the iris; and
a power component (208, 222, 242), operably coupled to the readout circuitry (209, 224, 244), configured to supply power to the readout circuitry (209, 224, 244).

2. The contact lens (102, 200, 210, 230) of claim 1, wherein the one or more light sensors (206, 214, 216, 218, 220, 234, 236, 238) comprise any of:
at least one of a complementary metal oxide semiconductor light sensor or a PN-junction light sensor;
at least one light sensor configured to detect selected light wavelengths;
at least one monochromatic light sensor; or
a circular array of two or more light sensors.

3. The contact lens (102, 200, 210, 230) of claim 1, wherein at least one of the one or more light sensors (206, 214, 216, 218, 220, 234, 236, 238) is less than approximately 500 microns wide.

4. The contact lens (102, 200, 210, 230) of claim 1, wherein the readout circuitry (209, 224, 244) is configured to output an iris fingerprint generated from the pattern, wherein the iris fingerprint is used for biometric identification.

5. The contact lens (102, 200, 210, 230) of claim 1, wherein the readout circuitry (209, 224, 244) is configured to:
output the information periodically;
output the information at least one of periodically in response to a polling signal or in response to an interrogation signal; or
output the information according to near field communication.

6. The contact lens (102, 200, 210, 230) of claim 1, wherein the power component (208, 222, 242) is configured to:
receive a signal according to near field communication;
receive power optically; or
receive power wirelessly, for example by comprising one or more radio frequency antennas configured to receive power via radio frequency communication.

7. A method facilitating biometric iris recognition, comprising:
detecting, at one or more light sensors (206, 214, 216, 218, 220, 234, 236, 238) disposed directly on or within a transparent contact lens (102, 200, 210, 230) covering at least a portion of an eye, light reflected from light incident on an iris (104) of the eye, wherein the one or more light sensors are configured to detect image data from the reflected light, the image data being indicative of a pattern associated with the iris (104); and
outputting an iris fingerprint based in part on the image data.

8. The method of claim 7, wherein the outputting the image data is performed according to a near field communication.

9. The method of claim 7, further comprising receiving an interrogation signal, and wherein the outputting the image data is performed in response to the receiving the interrogation signal.

10. The method of claim 7, wherein the light incident on the iris (104) of the eye comprises any of:
ambient light incident on the eye; or
a selected interrogation light spectra incident on the eye.

11. The method of claim 7, further comprising receiving power wirelessly at the one or more light sensors (206, 214, 216, 218, 220, 234, 236, 238).

12. A method of biometric identification, the method comprising:
employing logic circuitry (416) to execute computer executable components stored within a memory (414) to perform the following:
generating a first iris fingerprint based, at least, on an iris pattern retrieved via readout circuitry (209, 224, 244) communicatively coupled to one or more light sensors (206, 214, 216, 218, 220, 234, 236, 238) disposed directly on or within a contact lens (102, 200, 210, 230), wherein the one or more light sensors (206, 214, 216, 218, 220, 234, 236, 238) are configured to detect image data from light filtered by an iris (104) over which the contact lens (102, 200, 210, 230) is substantially positioned, the image data being indicative of a pattern associated with the iris;
comparing the first iris fingerprint with a second iris fingerprint; and
determining whether a biometric match exists between the first iris fingerprint and the second iris fingerprint based, at least, on the comparing.

13. The method of claim 12, wherein the one or more light sensors (206, 214, 216, 218, 220, 234, 236, 238) are at least one of a complementary metal oxide semiconductor light sensor or a PN-junction light sensor.

14. The method of claim 12, wherein retrieval via the readout circuitry is in response to polling the readout circuitry, wherein the polling is either periodic polling, or performed over a channel according to a near field communication.

15. A system facilitating biometric identification (402), the system comprising:
a memory (414) that stores a computer executable comparison component (406) and a computer executable biometric match component (410); and
logic circuitry (416) that executes the computer executable comparison and biometric components stored in the memory (414) to perform the method of claim 12.

16. The system of claim 15, further comprising a security component that unlocks a device based, at least, on a determination that the biometric match exists.

## Patentansprüche

1. Kontaktlinse (102, 200, 210, 230), die Folgendes umfasst:
ein transparentes Substrat (202, 212, 232), das ausgebildet ist, um zumindest einen Abschnitt einer Iris (104) eines Auges zu bedecken; und
eine Schaltung (204), die Folgendes umfasst:
einen oder mehrere Lichtsensoren (206, 214, 216, 218, 220, 234, 236, 238), die direkt auf dem oder innerhalb des transparenten Substrats (202, 212, 232) angeordnet sind und ausgelegt sind, um Licht zu detektieren, das von der Iris (104) reflektiert und somit durch diese gefiltert wird und auf den einen oder die mehreren Lichtsensoren (206, 214, 216, 218, 220, 234, 236, 238) auftrifft, wobei der eine oder die mehreren Lichtsensoren ausgelegt sind, um Bilddaten anhand des reflektierten Lichts zu detektieren, wobei die Bilddaten ein der Iris zugehöriges Muster angeben;
einen Ausleseschaltkreis (209, 224, 244), der mit dem einen oder den mehreren Lichtsensoren (206, 214, 216, 218, 220, 234, 236, 238) wirkverbunden und ausgelegt ist, um Informationen auszugeben, die das der Iris zugehörige Muster angeben; und
ein Leistungsbauteil (208, 222, 242), das mit dem Ausleseschaltkreis (209, 224, 244) wirkverbunden und ausgelegt ist, um die Ausleseschaltung (209, 224, 244) mit Leistung zu versorgen.

2. Kontaktlinse (102, 200, 210, 230) nach Anspruch 1, wobei der eine oder die mehreren Lichtsensoren (206, 214, 216, 218, 220, 234, 236, 238) einen beliebigen der nachstehenden umfassen:
zumindest einen aus einem Komplementärmetalloxidhalbleiterlichtsensor oder einem pn-Übergangslichtsensor;
zumindest einen Lichtsensor, der ausgelegt ist, um ausgewählte Lichtwellenlängen zu detektieren;
zumindest einen monochromatischen Lichtsensor oder
eine kreisförmige Anordnung von zwei oder mehr Lichtsensoren.

3. Kontaktlinse (102, 200, 210, 230) nach Anspruch 1, wobei zumindest einer des einen oder mehreren Lichtsensoren (206, 214, 216, 218, 220, 234, 236, 238) weniger als etwa 500 µm breit ist.

4. Kontaktlinse (102, 200, 210, 230) nach Anspruch 1, wobei der Ausleseschaltkreis (209, 224, 244) ausgelegt ist, um einen Iris-Abdruck, der anhand des Musters generiert wird, auszugeben, wobei der Iris-Abdruck zur biometrischen Identifikation verwendet wird.

5. Kontaktlinse (102, 200, 210, 230) nach Anspruch 1, wobei der Ausleseschaltkreis (209, 224, 244) ausgelegt ist, um:
die Informationen periodisch auszugeben;
die Informationen zumindest periodisch als Antwort auf ein Aufrufsignal oder als Antwort auf ein Abfragesignal auszugeben; oder
die Informationen gemäß Nahfeldkommunikation (NFC) auszugeben.

6. Kontaktlinse (102, 200, 210, 230) nach Anspruch 1, wobei das Leistungsbauteil (208, 222, 242) ausgelegt ist, um:
ein Signal gemäß NFC zu empfangen;
Leistung optisch zu empfangen; oder
Leistung drahtlos zu empfangen, beispielsweise indem es eine oder mehrere Hochfrequenzantennen umfasst, die ausgelegt sind, um Leistung über Hochfrequenzkommunikation zu empfangen.

7. Verfahren zur Ermöglichung von biometrischer Iriserkennung, wobei das Verfahren Folgendes umfasst:
das Detektieren an einem oder mehreren Lichtsensoren (206, 214, 216, 218, 220, 234, 236, 238), die direkt auf oder innerhalb einer transparenten Kontaktlinse (102, 200, 210, 230), die zumindest einen Abschnitt eines Auges bedeckt, angeordnet sind, von Licht, das von auf einer Iris (104) des Auges auftreffendem Licht reflektiert wird, wobei der eine oder die mehreren Lichtsensoren ausgelegt sind, um Bilddaten von dem reflektierten Licht zu detektieren, wobei die Bilddaten ein der Iris (104) zugehöriges Muster angeben; und
das Ausgeben eines Iris-Abdrucks teilweise auf Grundlage der Bilddaten.

8. Verfahren nach Anspruch 7, wobei das Ausgeben der Bilddaten gemäß einer Nahfeldkommunikation erfolgt.

9. Verfahren nach Anspruch 7, das ferner das Empfangen eines Abfragesignals umfasst, wobei das Ausgeben der Bilddaten als Antwort auf das Empfangen des Abfragesignals erfolgt.

10. Verfahren nach Anspruch 7, wobei das auf die Iris (104) des Auges auftreffende Licht ein beliebiges der nachstehenden umfasst:
Umgebungslicht, das auf das Auge auftrifft; oder
ein ausgewähltes Abfragelichtspektrum, das auf das Auge auftrifft.

11. Verfahren nach Anspruch 7, das ferner das drahtlose Empfangen von Leistung an dem einen oder den mehreren Lichtsensoren (206, 214, 216, 218, 220, 234, 236, 238) umfasst.

12. Verfahren zur biometrischen Identifikation, wobei das Verfahren Folgendes umfasst:
das Nutzen eines Logikschaltkreises (416) zum Ausführen von computerausführbaren Komponenten, die in einem Speicher (414) gespeichert sind, um Folgendes durchzuführen:
das Generieren eines ersten Iris-Abdrucks zumindest auf Grundlage eines Irismusters, das über den Ausleseschaltkreis (209, 224, 244) empfangen wurde, der mit einem oder mehreren Lichtsensoren (206, 214, 216, 218, 220, 234, 236, 238) kommunikativ gekoppelt ist, die direkt auf oder innerhalb einer Kontaktlinse (102, 200, 210, 230) angeordnet sind, wobei der eine oder die mehreren Lichtsensoren (206, 214, 216, 218, 220, 234, 236, 238) ausgelegt sind, um Bilddaten aus Licht zu detektieren, das durch eine Iris (104) gefiltert wird, über der die Kontaktlinse (102, 200, 210, 230) im Wesentlichen angeordnet ist, wobei die Bilddaten ein der Iris zugehöriges Muster angeben;
das Vergleichen des ersten Iris-Abdrucks mit einem zweiten Iris-Abdruck und
das Bestimmen auf Grundlage zumindest des Vergleichens, ob eine biometrische Übereinstimmung zwischen dem ersten Iris-Abdruck und dem zweiten Iris-Abdruck vorliegt.

13. Verfahren nach Anspruch 12, wobei der eine oder die mehreren Lichtsensoren (206, 214, 216, 218, 220, 234, 236, 238) zumindest einer aus einem Komplementärmetalloxidhalbleiterlichtsensor und einem pn-Übergangslichtsensor sind.

14. Verfahren nach Anspruch 12, wobei das Abrufen über den Ausleseschaltkreis als Antwort auf das Aufrufen der Ausleseschaltung erfolgt, wobei das Aufrufen entweder periodisches Aufrufen ist oder über einen Kanal gemäß einer Nahfeldkommunikation erfolgt.

15. System zur Ermöglichung biometrischer Identifikation (402), wobei das System Folgendes umfasst:
einen Speicher (414), der eine computerausführbare Vergleichskomponente (406) und eine computerausführbare biometrische Übereinstimmungskomponente (410) speichert; und
einen Logikschaltkreis (416), der die computerausführbaren Vergleichs- und biometrischen Komponenten, die in dem Speicher (414) gespeichert sind, ausführt, um ein Verfahren nach Anspruch 12 durchzuführen.

16. System nach Anspruch 15, das ferner eine Sicherheitskomponente umfasst, die eine Vorrichtung zumindest auf Grundlage einer Bestimmung, dass die biometrische Übereinstimmung vorliegt, entsperrt.

## Revendications

1. Lentille de contact (102, 200, 210, 230), comprenant : un substrat transparent (202, 212, 232) formé pour recouvrir au moins une partie d'un iris (104) d'un œil ; et
un circuit (204) comprenant :
un ou plusieurs capteurs de lumière (206, 214, 216, 218, 220, 234, 236, 238) disposés directement sur ou dans le substrat transparent (202, 212, 232) et configurés pour détecter la lumière réfléchie depuis, et donc filtrée à travers, l'iris (104) et incidente sur les un ou plusieurs capteurs de lumière (206, 214, 216, 218, 220, 234, 236, 238), dans laquelle les un ou plusieurs capteurs de lumière sont configurés pour détecter des données d'image à partir de la lumière réfléchie, les données d'image étant indicatives d'un motif associé à l'iris ;
des circuits de lecture (209, 224, 244), fonctionnellement couplés aux un ou plusieurs capteurs de lumière (206, 214, 216, 218, 220, 234, 236, 238), configurés pour délivrer en sortie des informations indicatives du motif associé à l'iris ; et
un composant d'alimentation (208, 222, 242), fonctionnellement couplé aux circuits de lecture (209, 224, 244), configuré pour distribuer de l'énergie aux circuits de lecture (209, 224, 244).

2. Lentille de contact (102, 200, 210, 230) selon la revendication 1, dans laquelle les un ou plusieurs capteurs de lumière (206, 214, 216, 218, 220, 234, 236, 238) comprennent l'un quelconque parmi :
au moins l'un parmi un capteur de lumière à semi-conducteur à base d'oxyde métallique complémentaire ou un capteur de lumière à jonction PN ;
au moins un capteur de lumière configuré pour détecter des longueurs d'onde de lumière sélectionnées ;
au moins un capteur de lumière monochromatique ; ou
un réseau circulaire de deux ou plus de deux capteurs de lumière.

3. Lentille de contact (102, 200, 210, 230) selon la revendication 1, dans laquelle au moins un des un ou plusieurs capteurs de lumière (206, 214, 216, 218, 220, 234, 236, 238) a une largeur inférieure à approximativement 500 microns.

4. Lentille de contact (102, 200, 210, 230) selon la revendication 1, dans laquelle les circuits de lecture (209, 224, 244) sont configurés pour délivrer en sortie une empreinte de l'iris générée à partir du motif, où l'empreinte de l'iris est utilisée pour une identification biométrique.

5. Lentille de contact (102, 200, 210, 230) selon la revendication 1, dans laquelle les circuits de lecture (209, 224, 244) sont configurés pour :
délivrer en sortie les informations périodiquement ;
délivrer en sortie les informations au moins l'un de périodiquement en réponse à un signal de scrutation ou en réponse à un signal d'interrogation ; ou
délivrer en sortie les informations selon une communication en champ proche.

6. Lentille de contact (102, 200, 210, 230) selon la revendication 1, dans laquelle le composant d'alimentation (208, 222, 242) est configuré pour :
recevoir un signal selon une communication en champ proche ;
recevoir de l'énergie optiquement ; ou
recevoir de l'énergie sans fil, par exemple en comprenant une ou plusieurs antennes à radiofréquence configurées pour recevoir de l'énergie par communication de radiofréquence.

7. Procédé permettant la reconnaissance biométrique de l'iris, comprenant : la détection, au niveau d'un ou plusieurs capteurs de lumière (206, 214, 216, 218, 220, 234, 236, 238) disposés directement sur ou dans une lentille de contact transparente (102, 200, 210, 230) recouvrant au moins une partie d'un œil, une lumière réfléchie à partir de lumière incidente sur un iris (104) de l'œil, dans lequel les un ou plusieurs capteurs de lumière sont configurés pour détecter des données d'image à partir de la lumière réfléchie, les données d'image étant indicatives d'un motif associé à l'iris (104) ; et
la délivrance en sortie d'une empreinte de l'iris basée en partie sur les données d'image.

8. Procédé selon la revendication 7, dans lequel la délivrance en sortie des données d'image est effectuée selon une communication en champ proche.

9. Procédé selon la revendication 7, comprenant en outre la réception d'un signal d'interrogation, et dans lequel la délivrance en sortie des données d'image est effectuée en réponse à la réception du signal d'interrogation.

10. Procédé selon la revendication 7, dans lequel la lumière incidente sur l'iris (104) de l'œil comprend l'une quelconque parmi :
une lumière ambiante incidente sur l'œil ; ou
un spectre de lumière d'interrogation sélectionnée incidente sur l'œil.

11. Procédé selon la revendication 7, comprenant en outre la réception d'énergie sans fil au niveau des un ou plusieurs capteurs de lumière (206, 214, 216, 218, 220, 234, 236, 238).

12. Procédé d'identification biométrique, le procédé comprenant :
l'utilisation de circuits logiques (416) pour exécuter des composants exécutables sur ordinateur stockés dans une mémoire (414) pour effectuer les actions suivantes :
la génération d'une première empreinte de l'iris sur la base, au moins, d'un motif d'iris extrait par l'intermédiaire de circuits de lecture (209, 224, 244) couplés en communication à un ou plusieurs capteurs de lumière (206, 214, 216, 218, 220, 234, 236, 238) disposés directement sur ou dans une lentille de contact (102, 200, 210, 230), dans lequel les un ou plusieurs capteurs de lumière (206, 214, 216, 218, 220, 234, 236, 238) sont configurés pour détecter des données d'image à partir de lumière filtrée par un iris (104) sur lequel la lentille de contact (102, 200, 210, 230) est sensiblement positionnée, les données d'image étant indicatives d'un motif associé à l'iris ;
la comparaison de la première empreinte de l'iris à une deuxième empreinte de l'iris ; et
la détermination du fait qu'il existe ou non une correspondance biométrique entre la première empreinte de l'iris et la deuxième empreinte de l'iris sur la base, au moins, de la comparaison.

13. Procédé selon la revendication 12, dans lequel les un ou plusieurs capteurs de lumière (206, 214, 216, 218, 220, 234, 236, 238) sont au moins l'un parmi un capteur de lumière à semi-conducteur à base d'oxyde métallique complémentaire ou un capteur de lumière à jonction PN.

14. Procédé selon la revendication 12, dans lequel l'extraction par l'intermédiaire des circuits de lecture est en réponse à la scrutation des circuits de lecture, dans lequel la scrutation est une scrutation périodique, ou effectuée sur un canal selon une communication en champ proche.

15. Système permettant une identification biométrique (402), le système comprenant :
une mémoire (414) qui stocke un composant de comparaison exécutable sur ordinateur (406) et un composant de mise en correspondance biométrique exécutable sur ordinateur (410) ; et
des circuits logiques (416) qui exécutent la comparaison exécutable sur ordinateur et
des composants biométriques stockés dans la mémoire (414) pour conduire le procédé selon la revendication 12.

16. Système selon la revendication 15, comprenant en outre un composant de sécurité qui déverrouille un dispositif sur la base, au moins, d'une détermination du fait que la correspondance biométrique existe.
